(19) Europäisches Patentamt
European Patent Office
Office européen des brevets

(11) EP 2 853 552 B1

(12) EUROPÄISCHE PATENTSCHRIFT

(45) Veröffentlichungstag und Bekanntmachung des Hinweises auf die Patenterteilung:
**06.12.2017 Patentblatt 2017/49**

(51) Int Cl.:
*C08G 63/78* (2006.01)     *C08L 67/04* (2006.01)
*C12P 7/56* (2006.01)      *G01N 33/487* (2006.01)
*C08G 63/08* (2006.01)     *C08G 63/89* (2006.01)
*C08G 63/90* (2006.01)     *C12N 1/02* (2006.01)
*G01N 33/04* (2006.01)     *G01N 33/44* (2006.01)

(21) Anmeldenummer: **14003051.1**

(22) Anmeldetag: **22.10.2012**

(54) **Verfahren zur Herstellung einer polymerisationsfähigen Milchsäure**

Method for producing a polymerizable lactic acid

Procédé de fabrication d'un acide lactique pouvant être polymérisé

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.11.2011 DE 102011117625**
**10.02.2012 DE 102012002498**

(43) Veröffentlichungstag der Anmeldung:
**01.04.2015 Patentblatt 2015/14**

(62) Dokumentnummer(n) der früheren Anmeldung(en) nach Art. 76 EPÜ:
**12786813.1 / 2 773 683**

(73) Patentinhaber: **Uhde Inventa Fischer GmbH**
**13509 Berlin (DE)**

(72) Erfinder:
• **Schulze, Joachim**
**59494 Soest (DE)**
• **Hagen, Rainer**
**13465 Berlin (DE)**
• **Tietz, Wolfgang**
**06406 Bernburg OT Biendorf (DE)**
• **Ghanegaonkar, Shashank**
**04109 Leipzig (DE)**
• **Mühlbauer, Udo**
**10119 Berlin (DE)**
• **Techlin, Willi**
**12347 Berlin (DE)**

(74) Vertreter: **Ossmann, Jens**
**ThyssenKrupp AG**
**ThyssenKrupp Allee 1**
**45143 Essen (DE)**

(56) Entgegenhaltungen:
**EP-A1- 2 031 009         EP-A1- 2 374 895**
**EP-A2- 0 411 780         WO-A2-01/81610**
**US-A1- 2002 132 967      US-A1- 2006 014 975**

• **SAARA INKINEN ET AL: "From Lactic Acid to Poly(lactic acid) (PLA): Characterization and Analysis of PLA and Its Precursors", BIOMACROMOLECULES, Bd. 12, Nr. 3, 14. März 2011 (2011-03-14), Seiten 523-532, XP055052721, ISSN: 1525-7797, DOI: 10.1021/bm101302t**

**Beschreibung**

Die Herstellung von Milchsäure aus kohlenhydrathaltigen Materialien durch Fermentation gewinnt zunehmend an Bedeutung. Dem Fachmann sind aber auch andere Möglichkeiten zur Gewinnung von Milchsäure über chemische Umwandlungen von Reaktanten, die aus der Petrochemie stammen, wie beispielsweise die Hydrolyse von Lactonitril, bekannt. Milchsäure ist ein umweltfreundliches Zwischenprodukt zur Herstellung von Reinigern, Flüssigseifen, Entkalkern und Textilhilfsmitteln. Das Interesse an Milchsäure ist in der letzten Zeit weiter gewachsen, weil die polymere Form der Milchsäure, das Polylactid, kompostierbar ist. Polylactid oder Polymilchsäure findet Anwendung als biologisch abbaubarer und gut verträglicher Kunststoff in der Lebensmittelindustrie, in der Kosmetik und in der Medizintechnik. Besonderes Interesse finden Tragetaschen aus kompostierbaren Polymilchsäurefolien, weil Tragetaschen aus konventionellem Kunststoff in der Umwelt nicht abgebaut werden und deshalb eine große Umweltbelastung darstellen. Plastiktragetaschen aus Polymilchsäure sind hingegen biologisch abbaubar und deshalb eine umweltfreundliche Alternative zu Tragetaschen aus konventionellem Kunststoff.

Milchsäure kommt in zwei diastereoisomeren Formen, als L(+)- und D(-)-Milchsäure vor. Ausgangsstoff zur fermentativen Herstellung von Milchsäure ist ein kohlenhydrathaltiges Material, das durch Umsetzung mit hierfür geeigneten Mikroorganismen in Milchsäure umgewandelt wird. Geeignete Bakterien hierfür sind beispielsweise Milchsäurebakterien vom Stamm der *Lactobacillaceae,* aber auch Mikroorganismen vom Stamm der *Saccharomyces* oder *Rhizopus.* Je nach eingesetztem Stamm der Mikroorganismen erhält man eine oder beide der oben genannten diastereoisomere Formen der Milchsäure.

Entscheidend für die industrielle Nutzung von Milchsäure, die durch Fermentation kohlehydrathaltiger Substrate mittels verschiedener Mikroorganismen erzeugt wird, ist die Wirtschaftlichkeit und Effizienz der Abtrennung und Reinigung der Milchsäure aus diesen wässrigen Fermentationslösungen, die neben der Milchsäure oder den Milchsäuresalzen auch weitere organische Säuren, sonstige Nebenprodukte der Fermentation, Mikroorganismen und deren Bestandteile sowie Reste der Substrate, wie Zucker, enthalten.

Diese Verunreinigungen stören bei einer anschließenden Polymerisation der Milchsäure zu Polymilchsäure und somit bei der Herstellung von biologisch abbaubaren Kunststoffen. Dass extrem reines Monomer eingesetzt werden muss, um einen hohen Polymerisationsgrad der Milchsäure zu erreichen, ist seit langem bekannt und geht beispielsweise aus J. Dahlmann et al, British Polymer Journal, Bd. 23 (1990), S. 235. 240 hervor.

Aus der Literatur ist eine Vielzahl von Methoden betreffend die Aufreinigung von Milchsäure bekannt.

Beispielsweise wird in einigen Patenten gelehrt, die Destillation zur Aufreinigung von Milchsäure aus wässrigen Lösungen zu nutzen. Ein derartiges Verfahren macht sich die EP 0986532 B2 zu nutze. In der DE 10 2007 045 701 B3 wird eine kombinierte Extraktion mit linearem n-Trioctylamin (TOA) und einer Destillation offenbart. Weitere in der Literatur bekannte Möglichkeiten sind die Elektrodialyse bzw. die Veresterung mit einem Alkohol, wonach ebenfalls eine Destillation und dann eine Hydrolyse des gebildeten Esters durchgeführt werden. Diese Verfahren sind äußerst kostenintensiv. Die Destillation bietet zudem den Nachteil, dass immer auch ein Teil der Kohlenhydrate mitextrahiert werden, was zu einer Verschlechterung der Ausbeute des gesamten Prozesses führt und die Isolierung des Produktes erschwert.

Auch Prozesse unter der Verwendung von Calciumhydroxid und Schwefelsäure, wobei als Nebenprodukt Gips in großen Mengen anfällt, sind bekannt. In diesem Zusammenhang wurde zudem gefunden, dass sich Milchsäure aus beispielsweise einer mit Schwefelsäure angesäuerten Fermentationsbrühe, die neben freier Milchsäure noch Ammonium- und Sulfationen enthält, mittels chromatographischer Methoden isolieren lässt. DE 69815369 T2 beschreibt beispielsweise unter anderem die Abtrennung von Milchsäure aus wässrigen Mischungen durch Adsorption an ein festes Adsorbens, vorzugsweise wird hier ein Feststoffadsorbens verwendet, das Milchsäure versus Lactat adsorbiert. Insbesondere kommen laut obiger Schrift schwache Anionenaustauscher zur Milchsäureisolierung in Frage. Die DE 10 2009 019 248 A1 beschreibt weiterhin chromatographische Methoden zur Aufreinigung organischer Säuren, im Speziellen von Milchsäure, indem eine Simulated Moving Bed Chromatographie durchgeführt wird.

Die WO 2006/124633 A1 beschreibt einen Prozess zur Herstellung von Ammoniumlactat durch Fermentation. Bei der Fermentation bildet sich das Ammoniumsalz der Milchsäure, das aus der Fermentationslösung z.B. durch Extraktion abgetrennt werden kann. Das Ammoniumsalz kann in einem Folgeschritt sehr leicht mit schwachen Säuren oder Kohlendioxid gespalten werden. Dabei gewinnt man die freie Milchsäure, die dann beispielsweise durch Destillation gereinigt werden EP2374895 A1 beschreibt einen Prozess zur Herstellung von Milchsäure, wobei Destillationsschritte zur Aufreinigung verwendet werden.

Nachteil vieler Verfahren ist, dass zusätzliche Stoffe dem Prozess zugeführt werden, die im Zielprodukt nicht mehr enthalten sein dürfen bzw. deren Spuren im Zielprodukt zu Einschränkungen in der Qualität und der Anwendbarkeit des Produktes führen können. So führen die Aufreinigungsverfahren aus dem Stand der Technik oft zu unzureichender Qualität der aufgereinigten Milchsäure und eine Polymerisation zu Polymilchsäure ist nicht im gewünschten Maße möglich. Auch ist die praktische Durchführung der Verfahren zum Teil mit erheblichem technischem und energetischem Aufwand verbunden. Die Qualität der aufgereinigten Milchsäure wird oft erst ersichtlich, wenn Polymilchsäure hergestellt werden soll.

Aufgabe der Erfindung ist es, ein Testverfahren zur Qualitätsbestimmung von Milchsäure bereitzustellen, das es vor der Herstellung von Polymilchsäure erlaubt, festzustellen, ob die vorliegende Milchsäure ein polymerisationsfähiges Material darstellt. Zudem soll ein Verfahren zur Herstellung von Milchsäuren aus Fermentationsbrühen zur Verfügung gestellt werden, das die Qualitätskriterien für ein polymerisationsfähiges Material erfüllt, die mit dem Test zur Qualitätsbestimmung ermittelt werden und bekannte Nachteile anderer Verfahren vermeidet.

Erfindungsgemäß wird die Aufgabe gelöst, durch den Einsatz eines Testverfahrens zur Qualitätsbestimmung von Milchsäure innerhalb des Verfahrens zur Herstellung von polymerisationsfähiger Milchsäure aus Fermentationsbrühen, umfassend die Verfahrensschritte

  a) Polykondensieren der Milchsäure zu einem Präpolymer
  b) Depolymerisieren zum Dilactid, und
  c) Bestimmung der Dilactidausbeute und der Racemisierung,

wobei Milchsäure, die den Test erfüllt und zur Polymerisation geeignet ist eine Dilactidausbeute von > 90 %, vorzugsweise < 93 % und eine Racemisierung von < 5 %, vorzugsweise <3 %, aufweist.

Dieser Test zur Qualitätsbestimmung erlaubt die Eignung einer Milchsäureprobe zur Herstellung von Polymilchsäure zu beurteilen. Die angegeben Dilacitdausbeuten und die Racemisierung von < 5 % sind zum Beispiel mit den in den Beispielen 1 und 2 beschriebenen Apparaturen und den dort beschriebenen verfahrenstechnischen Bedingungen zu erreichen. Der erfindungsgemäße Test besteht aus zwei Teilen: der Polykondensation der Milchsäure und der Depolymerisation des Polykondensats zu Lactid.

Mit Vorteil weist die in dem Test zur Qualitätsüberprüfung eingesetzte Milchsäure eine Dilactidausbeute von > 93 % auf. Des Weiteren ist es vorteilhaft, wenn die in dem Test zur Qualitätsüberprüfung eingesetzte Milchsäure eine Racemisierung von < 3 % aufweist. Dies sind die durch das Verfahren zur Qualtitätsbestimmung von Milchsäure bevorzugt zu erreichenden Kriterien, damit die Milchsäure für eine Polymerisation geeignet ist.

Bei der Polykondensation wird Milchsäure, die bevorzugt 88%ig bis 92%ig sein soll, über eine Zeitspanne von 5 bis 7 Stunden stufenweise von 120°C auf 180°C erhitzt und der Druck gleichzeitig von 350 mbar bis 450 mbar auf 100 mbar bis 25 mbar gesenkt. Des Weiteren wird in Verfahrensschritt a) nach einer Zeitspanne von 1 bis 3 Stunden bei einer Temperatur von 130°C bis 160°C und einem Druck von 150 mbar bis 250 mbar ein Katalysator zugesetzt, wobei der Katalysator bevorzugt Butylzinnoxid ist. Dabei wird durch stufenweises Erhöhen der Temperatur und gleichzeitiges Senken des Drucks innerhalb von sechs Stunden die zu testende Milchsäure zu einem Präpolymer polykondensiert.

Weiterhin wird das bei der Polykondensation erhaltene Präpolymer analytischen Verfahren zur Bestimmung der Molmasse zugeführt, wobei bevorzugt die Carboxylendgruppen bestimmt werden. Dabei wird das PLA-Oligomer (Poly Lactic Acid-Oligomer) in Aceton gelöst. Nach Zugabe von Methanol wird die Lösung mit 0,1 N benzylalkoholischer KOH-Lösung titriert. Der Endpunkt wird potentiometrisch erfasst. Aus der Carboxylendgruppenkonzentration ("COOH"), gemessen in mmol/kg, kann das Zahlenmittel der Molmasse berechnet werden nach der Gleichung

$$M_n = \frac{10^6}{COOH}.$$

Der Anstieg des Molekulargewichts über die Zeit ermöglicht bereits eine erste Abschätzung der Qualität der Milchsäure.

Die benötigten Mittel zur Polykondensation umfassen einen Dreihals-Rundkolben mit Rührer, Temperaturmeßfühler und aufgesetzter Rektifiziersäule. Der Kolben taucht in ein Ölbad ein, das mit einer Heizplatte erhitzt wird. Am Kopf der Rektifiziersäule ist ein Rückflußkühler angeordnet, der mit temperiertem Wasser auf 50°C bis 70°C gehalten wird. Oberhalb des Rückflußkühlers ist ein mit kaltem Wasser gekühlter Liebigkühler angeordnet, der in eine Vorlage mündet. Dort sammeln sich Nebenprodukte der Polykondensation wie Wasser. Begleitende Milchsäure wird in der Kolonne vom Wasser getrennt und fließt zurück in das Polykondensat. Über einen Seitenstutzen am Ausgang des Liebigkühlers wird eine Vakuumpumpe an die Apparatur angeschlossen. Das Vakuum wird mit Hilfe eines Nadelventils eingestellt. Bei der Kühlertemperatur nicht kondensierbare Bestandteile werden mit Hilfe einer beispielsweise mit Trockeneis gekühlten Kühlfalle vor der Vakuumpumpe abgeschieden. Generell gilt, dass jedwede dem Fachmann bekannte davon abweichende Konstruktion zur Polykondensation von Milchsäure denkbar ist.

Bei der Depolymerisation wird das bei der Polykondensation erhaltene Präpolymer, das sich im Dreihals-Rundkolben befindet, zunächst für 1,5 bis 2,5 Stunden bei einer Temperatur von 150°C bis 215°C und einem Druck von 180 bis 220 mbar gehalten und der Druck wird anschließend stufenweise auf einen Bereich von 50 bis 3 mbar abgesenkt. Dabei wird bevorzugt stündlich das aus dem Präpolymer gebildete Lactid gewogen, um das ermittelte Gewicht mit den Ergebnissen der in Verfahrensschritt c) durchzuführenden analytischen Methoden zu verrechnen.

Für die Depolymerisation zum Dilactid werden die beschriebenen Mittel zur Polykondensation so modifiziert, dass die Rektifiziersäule neben dem Dreihals-Rundkolben angeordnet und mit diesem über eine Rohrbrücke verbunden ist, so dass der Dampf aus dem Rundkolben von unten nach oben durch die Rektifiziersäule strömen kann und kein Rückfluß aus der Rektifiziersäule in den Dreihalskolben möglich ist. Das gebildete Dilactid entweicht dampfförmig aus dem Kolben, kondensiert im Rückflußkühler der Kolonne und sammelt sich in einem separaten Rundkolben, der die Kolonne nach unten abschließt. Nebenprodukte wie Wasser und Milchsäure kondensieren im Liebigkühler am Kopf der Rektifiziersäule und sammeln sich in der Vorlage.

Das in der Depolymerisation generierte Dilactid wird zur Bestimmung der Racemisierung und/oder des Dilactidumsatzes Mitteln zur Durchführung analytischer Methoden zugeführt, was z.B. über eine Trennung über eine HPLC und einer anschließenden Messung mittels eines UV-Detektors erfolgt. Auf diese Weise werden die Lactidausbeute (erzeugtes Lactid bezogen auf das eingesetzte Präpolymer) und die Enatiomerenreinheit des Lactids bestimmt. Dazu wird die Lactid-Probe in einem Gemisch aus 90/10 ml/ml n-Hexan/Ethanol gelöst. Die gelösten Komponenten werden mit HPLC auf einer chiralen Säule getrennt und mit einem UV-Detektor bei 223 nm analysiert. Daraus wird dann der Racemisierungsgrad und der Dilactidumsatz nach den nachstehenden Gleichungen berechnet:

(1) Berechung der Racemisierung: $Rac = \dfrac{\sum m_i \cdot (w_{i,meso} + w_{i,D})}{\sum m_i}$

- $m_i$: Masse der Lactidprobe i)
- $w_{i,meso}$: Massenanteil Mesolactid in der Lactidprobe i
- $w_{i,D}$: Massenanteil D-Lactid in der Lactidprobe i

(2) Berechung des Umsatzes: $Umsatz = \dfrac{\sum m_i}{m_{PP}}$

- $m_{PP}$: Gewicht des Präpolymers nach der Polykondensation

Gegenstand der vorliegenden Erfindung ist ein Verfahren zur Herstellung von polymerisationsfähiger Milchsäure aus Fermentationsbrühen, die eine Dilactidausbeute von > 90 % und eine Racemisierung von < 5 % aufweist. Diese Werte werden anhand des oben genannten Qualitätstests bestimmt und weisen eine Polymilchsäure aus, die sich zur Polymerisation eignet.

Das erfindungsgemäße Verfahren zur Herstellung von polymerisationsfähiger Milchsäure aus Fermentationsbrühen umfasst die Verfahrensschritte:- Abtrennen der Biomasse und eventuell vorhandener Feststoffe aus der Fermentationsbrühe in mindestens zwei aufeinanderfolgenden Stufen,- Absenken des pH-Wertes auf einen Wert von 2,2-2,4 durch Zugabe und Einmischen von konzentrierter Schwefelsäure in die Milchsäurelösung aus der biomassefreien Fermentationsbrühe,- Abtrennen von Milchsäurelösung aus der biomassefreien Fermentationsbrühe durch Simulated Moving Bed Chromatographie (SMB),- Reinigen der abgetrennten Milchsäurelösung mittels eines ersten Ionenaustauschschrittes,- Aufkonzentrieren der im ersten Ionenaustauschschritt aufgereinigten Milchsäurelösung mittels einer ersten ein- oder mehrstufigen Eindampfungsstufe,-weiteres Reinigen der vorkonzentrierten und vorgereinigten Milchsäurelösung mittels eines zweiten Ionenaustauschschrittes,- Aufkonzentrieren der im zweiten Ionenaustauschschritt aufgereinigten Milchsäurelösung mittels einer zweiten ein- oder mehrstufigen Eindampfungsstufe, so dass die Milchsäure eine Dilactidausbeute von > 90 % und eine Racemisierung von < 5 % aufweist; wobei die Herstellung der polymerisationsfähigen Milchsäure ohne Destillation erfolgt.

In bevorzugter Ausgestaltung des Verfahrens wird die erste Reinigung durch Ionentausch mit einer Nanofiltration kombiniert, wobei die Reinigung durch Ionenaustausch und Nanofiltration in beliebiger Reihenfolge angeordnet sind. Dadurch erfolgt eine effektive Feinreinigung.

Die Fermentationsbrühe, die die Milchsäure in Form von Ammoniumlactat, Biomasse und Bestandteile des Substrates enthält, wird kontinuierlich einer Precoat-Filtration und/oder einer Mikrofiltration und/oder einer Zentrifugation zugeführt. Dabei entsprechen Temperatur und pH-Wert den Werten der Fermentation, da festgestellt wurde, dass durch Inaktivierung der Biomasse durch Temperaturerhöhung und Absenken des pH-Wertes durch Zugabe von Säure eine Autolyse der Biomasse beschleunigt wird und mehr Lyseprodukte in die Fermentationsbrühe abgegeben werden. Auch sollte die Zeit zwischen Beendigung der Fermentation und der Abtrennung der Biomasse so kurz als möglich gehalten werden und sollte nicht mehr als 2 h betragen, und vorzugsweise weniger als 1 - 2 h betragen. Die Biomassekonzentration im Filtrat sollte 1 g/l nicht übersteigen. Durch diese Prozessführung wird die Endproduktqualität positiv beeinflusst.

Das Filtrat aus der Precoat- oder Mikrofiltration wird im ersten Verfahrensschritt einer zweiten Stufe zugeführt, die von einer ein- oder zweistufigen Ultrafiltration gebildet wird. Hier werden restliche Biomasseanteile, unlösliche Feststoffe und höhermolekulare Verbindungen abgetrennt. Als Optimum zwischen Produktqualität und Fluxraten der Membranen wurden Membranen mit einer Trenngrenze von ≤10 kDa ermittelt. Die Temperatur der flüssigen Medien sollte wegen des Löslichkeitskoeffizienten von Ammoniumlaktat in Wasser ≥ 30 °C betragen. Das Retentat wird zur Precoat- oder Mikrofiltration zurückgeführt. Das Permeat wird den weiteren Verfahrensschritten zugeführt.

Im Permeat der Ultrafiltration liegt die Milchsäure in Form ihres Salzes als Ammoniumlaktat vor. Zur Überführung in die Milchsäure erfolgt die Zugabe und Einmischung von konzentrierter Schwefelsäure und damit verbunden eine Absenkung des pH-Wertes der Lösung auf Werte zwischen 2,2 bis 2,4. Dabei wird die Ansäuerung in zwei Schritten durchgeführt, wobei in einem 1. Schritt die Fermentationsbrühe aus der ersten Stufe des ersten Verfahrensschrittes mit konzentrierter Schwefelsäure auf einen pH-Wert von 4,4 bis 4,6 angesäuert wird, und in einem 2. Schritt das Permeat der Ultrafiltration aus der zweiten Stufe des ersten Verfahrensschrittes mit konzentrierter Schwefelsäure auf einen pH-Wert von 2,0 bis 2,4 angesäuert wird, somit das in der gereinigten Fermentationslösung enthaltene Salz der Milchsäure in Milchsäure überführt wird und im stöchiometrischen Verhältnis Ammoniumsulfat entsteht. Zur Vermeidung von unerwünschter Ausfällung erfolgt dieser Prozessschritt bei Temperaturen zwischen 30°C bis 60°C und vorzugsweise in einem Bereich zwischen 30°C bis 40°C. Diese vorgereinigte Lösung steht für die Abtrennung und Reinigung der Milchsäure zur Verfügung.

Die Trennung des sauren Permeates der Ultrafiltration erfolgt in einer Simulated Moving Bed Chromatographie. Diese stellt eine besonders leistungsfähige Variante der High Performance Liquid Chromatographie dar, wobei durch die Aufeinanderfolge von mehreren über Ventile miteinander verbundenen Trennsäulen in einer Endlosschleife eine große Anzahl theoretischer Böden verwirklicht und die Trennschärfe der Chromatographie erheblich verbessert wird. Als stationäre Phase kommen Kationenaustauscher und Anionenaustauscher zum Einsatz. Auf Grund der unterschiedlichen Retentionszeiten für Milchsäure und Ammoniumsulfat kommt es zur Trennung der beiden Hauptkomponenten. Als Eluent kommen entmineralisiertes Wasser und/oder Brüdenkondensat zur Verwendung. Es konnte gezeigt werden, dass im Extrakt mehr als 95 % der im Permeat der Ultrafiltration enthaltenen Milchsäure gewonnen werden kann, wobei das Verhältnis zwischen Permeat der Ultrafiltration und Eluent im Bereich zwischen 1 : 1 und 1 : 2,5 variiert und acht in einer Endlosschleife geschaltete Anionenaustauscher-Säulen Verwendung fanden. Der Extrakt enthält nur noch geringe Mengen an Ammoniumsulfat, Essigsäure und Farbstoffe aus der Fermenterbrühe. Das ausgespülte Raffinat enthält maximal 1 g/l Milchsäure sowie das Ammoniumsulfat, Begleitsalze aus der Fermentation wie Phosphate, Nitrate und Chloride.

Um die Herstellung von Milchsäure in hochreiner und polymerisationsfähiger Qualität zu erzeugen, wird aufgrund noch vorhandener Reste von Farbstoffen und Begleitstoffen im nächsten Verfahrensschritt eine zweistufige Reinigung durch Ionenaustausch und Nanofiltration der SMB nachgeschaltet. Dabei können diese beiden Stufen in beliebiger Reihenfolge angeordnet werden. Als Ionenaustauscherharze kommen in Abhängigkeit von der chemischen Analyse der Verunreinigungen Kationen- und/oder Anionenaustauscher in Betracht. Dabei werden die gleichen Anionen- oder Kationenaustauscherharze wie bei der SMB verwendet, oder alternativ werden zur SMB unterschiedliche Ionenaustauscherharze verwendet. Die Nanofiltration dient vor allem der Feinreinigung des Extraktes aus den vorherigen Verfahrensschritten, wobei die Membranen eine Trenngröße von 100 bis 400 Da aufweisen. Es konnte gezeigt werden, dass eine Nanofiltration mit einer Trenngrenze um 200 Da gute Qualitätsergebnisse ergibt. Dabei wird der Prozess so geführt, dass das Retentat der Nanofiltration nicht mehr als 10% des Gesamtdurchsatzes beträgt. Das Permeat wird den weiteren Verfahrensschritten zugeführt.

In weiterer Ausgestaltung des Aufreinigungsverfahrens wird das Permeat aus diesem Verfahrensschritt einer ersten ein- oder mehrstufigen Eindampfung zugeführt. Dabei erfolgt eine Eindampfung auf eine Konzentration von 45 bis 55 Ma-% Milchsäure.

Die daraus resultierende vorkonzentrierte und vorgereinigte Milchsäurelösung wird dann im nächsten Verfahrensschritt abermals einem Ionenaustausch unterzogen. Auch hier gilt, dass im Vergleich zu den vorherigen Ionenaustauschschritten gleiche oder dazu unterschiedliche Chromatographieharze eingesetzt werden.

Im letzten Verfahrensschritt des erfindungsgemäßen Verfahrens wird dann die aufgereinigte Milchsäurelösung aus dem vorigen Verfahrensschritt auf eine Milchsäurekonzentration von ≥ 80 Ma-% aufkonzentriert, bevorzugt von 88 bis 92 Ma-%.

Gegenstand der vorliegenden Erfindung ist auch die Verwendung der aufgearbeiteten Milchsäure nach dem Verfahren der Ansprüche 1 bis 13 zur Herstellung von Polylactiden.

Weiterhin wird die gemäß dem Verfahren nach den Ansprüchen 1 bis 13 hergestellte Milchsäure zur Herstellung von Polylactiden verwendet. Dabei umfasst das Verfahren zur Herstellung von Polylactiden bevorzugt die nachfolgenden Verfahrensschritte:

- Aufkonzentrieren der polymerisationsfähigen Milchsäure auf ≥ 98 Ma-%,

- Präkondensation der aufkonzentrierten Milchsäure auf eine mittlere Molmasse von 500 bis 2000 g/mol,

- Cyclisierende Depolymerisation der präkondensierten Milchsäure, wobei das Lactid generiert wird,

- Reinigung des Lactids,

- Ringöffnungspolymerisation des Lactids, wobei Polylactid generiert wird,

- Entmonomerisierung des Polylactids,

- Granulierung des Polylactids zu Polylactid Granulat und Kristallisation des Polylactid Granulats.

Nachfolgend soll die vorliegende Erfindung anhand von Beispielen und Figuren näher erläutert werden.

Figur 1.: Schematischer Aufbau einer Vorrichtung zur Polykondensation der Milchsäure, die zur Durchführung des Tests zur Qualitätsbestimmung der Milchsäure geeignet ist.

Figur 2.: Schematischer Aufbau einer Vorrichtung zur Depolymerisation des Polykondensats der Polykondensation, die zur Durchführung des Tests zur Qualitätsbestimmung der Milchsäure geeignet ist.

Beispiel 1

Polykondensation

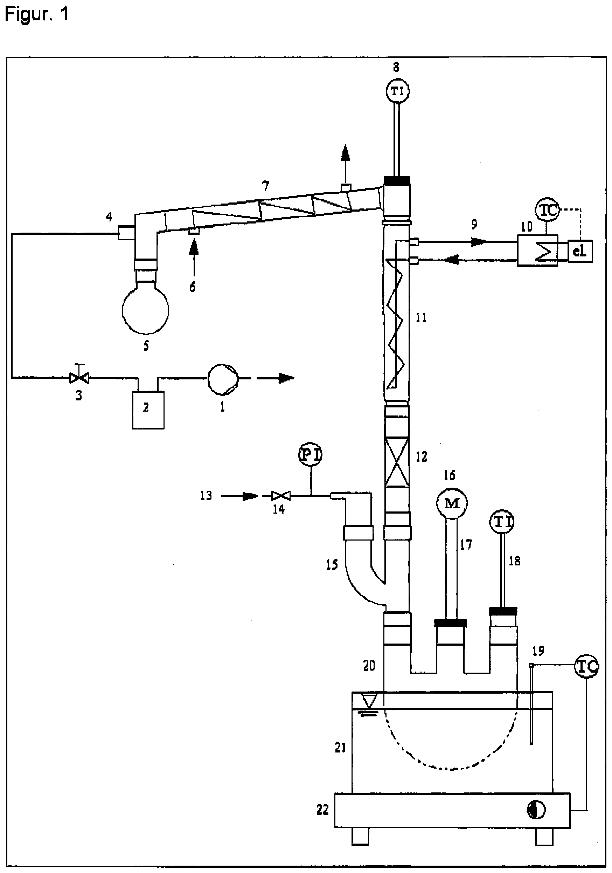

Die Polykondensation wird anhand der in Fig. 1 exemplarisch dargestellten Vorrichtung durchgeführt. Ein Dreihalsrundkolben 20 taucht in ein Ölbad 21 ein, das mit einer Heizplatte 22 erhitzt wird. Der Dreihalsrundkolben 20 wird vor dem Eintauchen in das Ölbad 21 gewogen und mit einer abgewogenen Menge an Milchsäure beschickt. Ein mit dem Dreihalsrundkolben verbundener Zweihalsaufsatz 15 dient der Zuführung von Stickstoff 13 über das Ventil 14. Ein auf den Zweihalsaufsatz 15 aufgesetzter Rückflußkühler 11 wird mittels eines Wärmeträgerablaufs 9 und einem Thermostat 10 mit temperiertem Wasser auf 50°C bis 70°C gehalten. Oberhalb des Rückflußkühlers 11 ist ein mit kaltem Wasser 6 gekühlter Liebigkühler 7 angeordnet, der in eine Vorlage 5 mündet. Dort sammeln sich Nebenprodukte der Polykondensation wie Wasser. Begleitende Milchsäure wird in der Rektifiziersäule 12 vom Wasser getrennt und fließt zurück in das Polykondensat im Dreihalsrundkolben 20. Über einen Seitenstutzen 4 am Ausgang des Liebigkühlers 7 wird eine Vakuumpumpe 1 an die Apparatur angeschlossen. Das Vakuum wird mit Hilfe eines Nadelventils eingestellt. Bei der Kühlertemperatur nicht kondensierbare Bestandteile werden mit Hilfe einer beispielsweise mit Trockeneis gekühlten Kühlfalle 2, die mit einem Ventil 3 in Verbindung steht, vor der Vakuumpumpe abgeschieden. Zur Temperaturüberwachung des Prozesses sind verschiedene Thermometer 8, 18 und 19 vorgesehen. Nach dem Eintauchen des Dreihalsrundkolbens 20 in das Ölbad 21 wird dessen Temperatur über eine Zeitspanne von 6 Stunden stufenweise von 120°C auf 180°C erhöht und der Druck von 400 auf 50 mbar gesenkt. Dabei wird mittels des Rührwerks 16 und der Rührwelle 17 gerührt. Nach zwei Stunden, bei einer Badtemperatur von 150°C und einem Druck von 200 mbar, wird die erste Probe genommen und der Katalysator dosiert. Als Katalysator wird Butylzinnoxid (TW30 der Firma Acima, gelöst in einer Mischung aus Butandiol und Methylisobutylketon) verwendet, wobei die Konzentration 500 ppm elementares Zinn bezogen auf die Masse des Präpolymers beträgt. Weitere Proben vom Präpolymer werden nach 4 h Stunden (180°C, 200 mbar) und nach 6 h (Versuchsende, 180°C, 50 mbar) genommen.

Beispiel 2

Depolymerisation

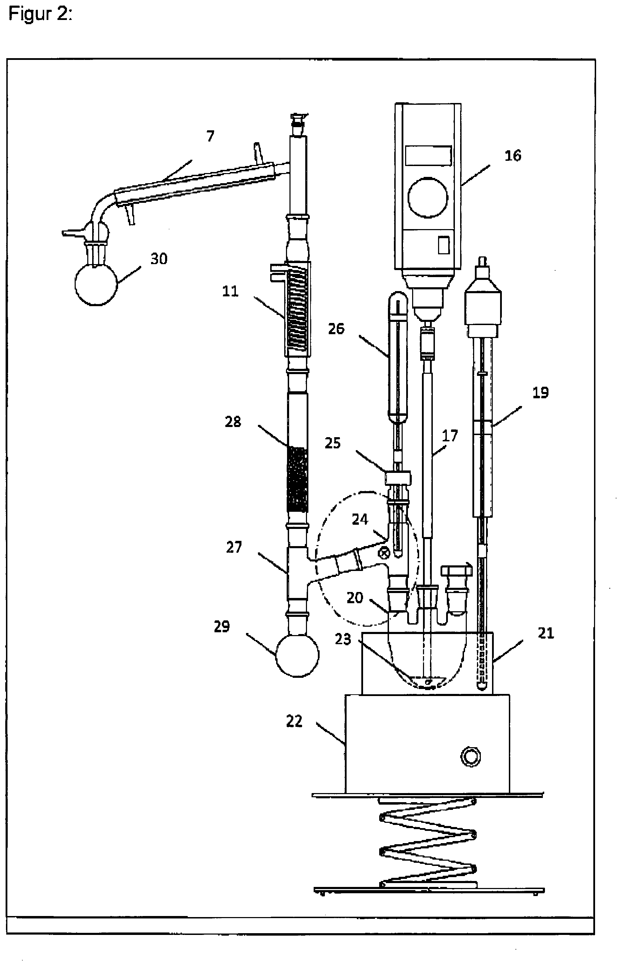

Für die Depolymerisation bleibt das Präpolymer im Dreihalsrundkolben 20 und der Aufbau wird derart modifiziert, dass die Rektifiziersäule 28+11 neben dem Dreihalsrundkolben 20 angeordnet und mit diesem über eine Rohrbrücke verbunden ist, so dass der Dampf aus dem Rundkolben von unten nach oben durch die Rektifiziersäule 28+11 strömen kann und kein Rückfluß aus der Rektifiziersäule in den Dreihalskolben möglich ist. Dies ist in Fig. 2 dargestellt. Dazu ist der Aufbau auf dem Dreihalskolben wie folgt. Der Dreihalsrundkolben 20 ist über einen ersten Destillieraufsatz 24 und einem Gewinderohr mit Schraubverschluss 25 mit einem Thermometer 26 verbunden. Der erste Destillieraufsatz 24 steht in Verbindung mit einem zweiten Destillieraufsatz 27, an dessen unterem Ende ein Hohlglasstutzen 29 angebracht ist. Oberhalb des zweiten Destillieraufsatzes 27 befindet sich eine Füllkörperkolonne 28 und ein Rückflußkühler 11. Oberhalb des Rückflußkühlers 11 ist ein Liebigkühler 7 angeordnet, der in einen weiteren Hohlglasstutzen 30 mündet. Bei der Reaktion im Dreihalsrundkolben 20 wird kontinuierlich gerührt. Dies erfolgt über das Rührwerk 16, das über die Rührwelle 17 mit dem Rührer 23 verbunden ist. Das gebildete Dilactid entweicht dampfförmig aus dem Dreihalsrundkolben 20, kondensiert im Rückflußkühler 11 der Kolonne und sammelt sich in dem separaten Hohlglasstutzen 29, der die Kolonne nach unten abschließt. Nebenprodukte wie Wasser und Milchsäure kondensieren im Liebigkühler 7 am Kopf der Rektifiziersäule und sammeln sich im Hohlglasstutzen 30. Der Dreihalsrundkolben 20 wird dazu in ein auf 210°C erhitztes Ölbad 21 getaucht, der Druck auf 200 mbar eingestellt und der Rührer 23 gestartet. Die Badtemperatur wird nach 115 min auf 220°C erhöht und dort gehalten. Der Druck wird nach 30 min auf 50 mbar, nach 45 min auf 10 mbar und nach 165 min auf 5 mbar gesenkt. Nach 75 min wird die Apparatur belüftet und der Hohlglasstutzen 29, in dem sich das gebildete Lactid sammelt, gewechselt. Ein weiterer Wechsel erfolgt nach 135 min, so dass insgesamt 3 Lactidproben entstehen. Die Temperaturen in der Apparatur werden über verschiedene Thermometer 19, 26 überwacht.

Beispiel 3

Abtrennung von Biomasse und Feststoffen bei dem erfindungsgemäßen Verfahren zur Herstellung von polymerisationsfähiger Milchsäure

Eine Fermenterbrühe mit einem pH-Wert von 6,8 und einem Gehalt an Ammoniumlaktat von 12,2 Ma% berechnet als Milchsäure wird zur Abtrennung der Biomasse über einen Separator gegeben.

Der Gehalt an Trockensubstanz (TS) in der unbehandelten Brühe lag bei 6,9 g/l. Nach dem Separator wurde im Klarlauf ein Gehalt an TS von < 0,03 g/l gemessen. Der Feststoffgehalt in der abgetrennten Biomasse lag bei 34,7 Ma%. Der Separator wurde mit ca. 360 l/h gefahren.

Erfindungsgemäß erfolgte die Ansäuerung der Fermenterbrühe mit Schwefelsäure in zwei Stufen, auf pH 4,5 vor und auf pH 2,2 nach der Ultrafiltration.

Die Ultrafiltration wurde über ein Wickelmodul durchgeführt. Die Membrane bestand aus Polyethersulfonat (PES) mit einer Fläche von 7 m$^2$.

Dank der guten Vorklärung im Separator wurden sehr hohe Fluxwerte erreicht, die im Versuchszeitraum praktisch konstant blieben. Das Retentat wies eine sehr dunkle Farbe auf und wurde zur Vermeidung von Milchsäureverlusten einer Diafiltration unterzogen.

Das Permeat wurde nach Ansäuerung auf pH 2,2 der SMB zugeführt.

Beispiel 4

SMB mit Anionentauscherharz bei dem erfindungsgemäßen Verfahren zur Herstellung von polymerisationsfähiger Milchsäure

Die nach dem erfindungsgemäßen Verfahren wie in Beispiel 3 gereinigte und angesäuerte Fermenterbrühe wurde über eine SMB zur Trennung von Milchsäure und Ammoniumsulfat gefahren. Als Eluent diente Deionat.

Die Säulen der SMB - Anlage waren mit einem mit Laktat beladenen Anionentauscherharz befüllt. Tabelle 1 zeigt die Ergebnisse der SMB:

Tabelle 1

| Ion | Ausgangswert | Extrakt | Raffinat | Wirkungsgrad |
|---|---|---|---|---|
| Sulfat | 75.084 mg/l | 650,3 mg/l | 34.697 mg/l | 98,34 % |
| Laktat | 117,4 g/l | 70,2 g/l | 1,44 g/l | 97,77 % |
| Menge | - | 1,8 l | 2,0 l | - |

Beispiel 5

SMB mit Kationentauscherharz bei dem erfindungsgemäßen Verfahren zur Herstellung von polymerisationsfähiger Milchsäure

In der gleichen SMB - Anlage, jedoch mit einer Füllung von Kationentauscherharz, das mit Ammoniumionen beladen war, wurden folgende Werte gemessen:

Tabelle 2

| Ion | Ausgangswert | Extrakt | Raffinat | Wirkungsgrad |
|---|---|---|---|---|
| Sulfat | 71.580 mg/l | 190 mg/l | 46.920 mg/l | 99,54 % |
| Laktat | 113,02 g/l | 74,26 g/l | 0,55 g/l | 99,34 % |

Die Werte zeigen, dass die Trennwirkung am Kationentauscherharz noch besser als in Beispiel 4 ist.

Das Sulfat wird zu 99,54% eliminiert und 99,34 % der Milchsäure gelangen in den Extrakt.

Beispiel 6

Nanofiltration bei dem erfindungsgemäßen Verfahren zur Herstellung von polymerisationsfähiger Milchsäure

In einer Laboranlage bestückt mit einer 0,07 m$^2$ Flachmembran 250 Dalton wurde der Effekt der Nanofiltration auf die erfindungsgemäß hergestellte Milchsäurelösung, die als Extrakt aus der SMB kam, getestet. Die Lösung hatte etwa 50°C. Der Druck vor der Membran wurde auf 30 bar eingestellt.

Das Permeat zeigt eine deutlich hellere Farbe, der Gehalt an Zucker, gemessen wurde Maltose, verringert sich stark und auch der Sulfatgehalt wird reduziert. Wesentlich ist auch die Reduzierung der Aminosäuren.

Tabelle 3 zeigt Messwerte zu den genannten Effekten:

Tabelle 3

| Parameter | Ausgangswert | Permeat |
|---|---|---|
| Yellowness Index (YI) | 28,55 | 2,4 |
| Maltose g/l | 1,1 | 0,05 |
| Sulfat g/l | 0,26 | 0,05 |
| Alanin | 0,22 | 0,10 |
| Asparaginsäure | 1,23 | 0,35 |

Zur weiteren Entfernung von Verunreinigungen wurde die Milchsäurelösung über eine Kombination von Entfärbersäule, Kationen- und Anionentauschersäule geleitet.

Beispiel 7

Eindampfung, gefolgt von Ionentausch und nochmaliger Eindampfung bei dem erfindungsgemäßen Verfahren zur Herstellung von polymerisationsfähiger Milchsäure

Die erfindungsgemäß hergestellte Milchsäurelösung enthält nach der zweistufigen Reinigung durch Nanofiltration und Ionenaustausch noch eine Reihe von Ionen, geringe Mengen Restzucker und färbende Substanzen.

Zur nun möglichst vollständigen Entfernung aller Verunreinigungen wird die Milchsäurelösung über eine Kombination von Entfärbersäule, Kationen- und Anionentauschersäule geleitet, auf ca. 50 Ma% Milchsäure eingedampft und erneut über die genannte Kombination von Entfärber- und Ionentauschersäulen geleitet.

Nach Durchlauf der oben beschriebenen Anordnung wurde eine 61 Ma% Milchsäure mit folgenden Parametern erhalten:

Sulfat: 2,6 mg/l

Phosphat: 0,23 mg/l

Maltose: 0,04 g/l

YI: 0,72

Die Milchsäurelösung war auch nach Durchlaufen der zweiten Eindampfungsstufe, wobei eine Milchsäurekonzentration von 90 Ma% Milchsäure erzielt wurde, wasserklar. Die derart aufgereinigte Milchsäure erfüllt den erfindungsgemäßen Qualitätstest und weist eine Lactidausbeute von 91,7% und eine Racemisierung von 3,3% auf und eignet sich somit als Ausgangsprodukt zur PLA-Herstellung.

Beispiel 8

Beschreibung eines Verfahrens zur Herstellung von Polylactiden, das die Milchsäure verwendet, die durch das erfindungsgemäße Verfahren zur Milchsäureherstellung generiert wurde

Bevorzugt wird dabei auf ein Verfahren Bezug genommen, das in der WO2009/030397 A1 detailliert offenbart ist.

a) Aufkonzentration von Milchsäure

Als Ausgangsmaterial dient Milchsäure, wie sie das erfindungsgemäße Verfahren nach einem der Ansprüche 1 bis 13 liefert. Die Trennung von Wasser und Milchsäure wird dabei in einer Rektifikationssäule vorgenommen. Dabei wird über einen Absaugstutzen Vakuum angelegt und das dampfförmig anfallende Wasser wird kondensiert und über einen weiteren Stutzen kopfseitig entnommen. Die Zuführung der Milchsäure erfolgt dabei kontinuierlich. Das Destillat ist reines Wasser, das sumpfseitig anfallende Produkt ist Milchsäure mit einer Konzentration von mehr als 99 Gew.-%.

b) Präkondensation

Die aufkonzentrierte Milchsäure wird in einer Serie von zwei Reaktoren durch Polykondensation in ein Präpolymer überführt. Die Polykondensation läuft unter zwei verschiedenen Drücken und Temperaturen ab, um den Reaktionsumsatz zu optimieren. Im ersten Reaktor sind die Konditionen so gewählt, dass die Verdampfung von Milchsäure minimiert ist und gleichzeitig die Entfernung von Wasser erleichtert wird. Im zweiten Schritt der Polykondensation ist die Reaktionsgeschwindigkeit durch eine höhere Temperatur erhöht, gleichzeitig wird der Druck vermindert, um die Wasserkonzentration in der Schmelze weiter zu mindern. Die mittlere Molmasse (Zahlenmittel) des Präpolymers liegt dabei zwischen 500 und 2.000 g/mol.

c) Cyclisierende Depolymerisation

Das Präpolymer steht in chemischem Gleichgewicht mit dem cyclischen Dimer der Milchsäure, dem Dilactid. Durch Einstellung von Druck und Temperatur im Depolymerisationsreaktor ist gewährleistet, dass das Lactid kontinuierlich aus dem Präpolymer gebildet wird und verdampft. Zum Depolmerisationsreaktor gehört ein Kondensator, der die Reaktionsbrüden teilweise kondensiert: Wasser und der größte Anteil an Milchsäure bleiben dabei dampfförmig und werden in einer Kondensationsvorrichtung wiederum teilweise kondensiert. Das Kondensat aus dem Depolymerisationsreaktor enthält zuvorderst das Lactid, Lactoylmilchsäure (das lineare Dimer der Milchsäure) und höhere lineare Oligomere.

d) Lactid-Reinigung

Während der Ringöffnungspolymerisation hängt das erreichbare Molekulargewicht und somit bedeutende mechanische Eigenschaften des Polylactids vom Reinheitsgrad des Lactids ab. Die Hydroxyl-Gruppen der als Verunreinigung enthaltenen Milchsäure und Lactoylmilchsäure dienen dabei als Ausgangspunkt der Polymerisation. Je höher die Konzentration der Hydroxyl-Gruppen im Lactid ist, desto geringer fällt das erreichbare Molekulargewicht des Polymers aus. Die Konzentration der Hydroxyl-Gruppen im Rohlactid ist nach der cyclisierenden Depolymerisation zu hoch. Das kondensierte Lactid wird in einer Rektifikationssäule bis zur benötigten Hydroxylgruppenkonzentration aufgereinigt. Das gereinigte Lactid wird der Säule als Seitenstrom entnommen. Das Destillat und das Sumpfprodukt können dem Prozess an unterschiedlichen Stellen wieder zugeführt werden.

e) Ringöffnungspolymerisation

Die Ringöffnungspolymerisation wird in einem Reaktor unternommen, der aus einer Kombination eines Rührkessels und eines Rohrreaktors gebildet ist. Im ersten Reaktor wird das niedrigviskose Lactid mit einer Umsetzungsrate von ca. 50 - 70 % zu PLA polymerisiert. Katalysator und Additive werden homogen in die Schmelze eingemischt. Im Rohreaktor wird die Polymerisationsreaktion so lange fortgeführt, bis ein chemisches Gleichgewicht zwischen Polymer und Monomer erreicht wird. Die maximale Umsetzung des Monomers beträgt ca. 95 %.

e) Entmonomerisierung

Um ein stabiles Polylactid zu erhalten, ist die Monomerkonzentration von ungefähr 5 Gew.-% in der Schmelze zu hoch. Deswegen muss eine Entmonomerisierung durchgeführt werden. Dies wird durch eine Entgasung der Schmelze z.B. in einem Doppelschneckenextruder erreicht. Aufgrund der Tatsache, dass die Ringöffnungspolymerisation eine Gleichgewichtsreaktion ist, wird ein Stabilisator vor der Entmonomerisierung zugegeben, um die Rückbildung des Monomers während und nach der Entgasung zu verhindern.

f) Granulierung und Kristallisation

Anschließend an die Entmonomerisierung wird die Schmelze dem Entmonomerisierungsapparat entnommen und in ein Granulat überführt. Dabei können sowohl Stranggranulation als auch Unterwasser-Heißabschlag-Granulation durchgeführt werden. In beiden Fällen muss das PLA-Granulat vor der Trocknung und der Verpackung kristallisiert werden. Die Kristallisation wird bei erhöhten Temperaturen und unter Rühren durchgeführt.

Bezugszeichenliste

| | |
|---|---|
| 1 | Vakuumpumpe |
| 2 | Kühlfalle |
| 3 | Ventil |
| 4 | Seitenstutzen |
| 5 | Vorlage |
| 6 | kaltes Wasser |
| 7 | Liebigkühler |
| 8 | Thermometer |
| 9 | Wärmeträgerablauf |
| 10 | Thermostat |
| 11 | Intensivkühler |
| 12 | Kolonne |
| 13 | Stickstoffzuleitung |
| 14 | Ventil |
| 15 | Zweihalsaufsatz |
| 16 | Rührwerk |
| 17 | Rührwelle |
| 18 | Thermometer |
| 19 | Thermometer |
| 20 | Dreihalsrundkolben |
| 21 | Ölbad |
| 22 | Heizplatte |
| 23 | Rührer |
| 24 | erster Destillieraufsatz |
| 25 | Gewinderohr mit Schraubverschluss |
| 26 | Thermometer |
| 27 | zweiter Destillieraufsatz |
| 28 | Füllkörperkolonne |
| 29 | Hohlglasstutzen |
| 30 | weiterer Hohlglasstutzen |

**Patentansprüche**

1. Verfahren zur Herstellung von polymerisationsfähiger Milchsäure aus Fermentationsbrühen, umfassend die Verfahrensschritte:

- Abtrennen der Biomasse und eventuell vorhandener Feststoffe aus der Fermentationsbrühe in mindestens zwei aufeinanderfolgenden Stufen,
- Absenken des pH-Wertes auf einen Wert von 2,2-2,4 durch Zugabe und Einmischen von konzentrierter Schwefelsäure in die Milchsäurelösung aus der biomassefreien Fermentationsbrühe,
- Abtrennen von Milchsäurelösung aus der biomassefreien Fermentationsbrühe durch Simulated Moving Bed Chromatographie (SMB),

- Reinigen der abgetrennten Milchsäurelösung mittels eines ersten Ionenaustauschschrittes,
- Aufkonzentrieren der im ersten Ionenaustauschschritt aufgereinigten Milchsäurelösung mittels einer ersten ein- oder mehrstufigen Eindampfungsstufe,
- weiteres Reinigen der vorkonzentrierten und vorgereinigten Milchsäurelosung mittels eines zweiten Ionenaustauschschrittes,
- Aufkonzentrieren der im zweiten Ionenaustauschschritt aufgereinigten Milchsäurelösung mittels einer zweiten ein- oder mehrstufigen Eindampfungsstufe, so dass

die Milchsäure eine Dilactidausbeute von > 90 % und eine Racemisierung von < 5 % aufweist; wobei die Herstellung der polymerisationsfähigen Milchsäure ohne Destillation erfolgt.

2. Verfahren nach Anspruch 1, wobei die Milchsäure einem Testverfahren unterzogen wird umfassend die Verfahrensschritte:

- Polykondensieren der Milchsäure zu einem Präpolymer,
- Depolymerisieren des Präpolymers zum Dilactid, und
- Bestimmen der Dilactidausbeute und der Racemisierung, wobei Milchsäure, die eine Dilactidausbeute von > 90 %, vorzugsweise > 93 % und eine Racemisierung von < 5 %, vorzugsweise < 3 % aufweist, eine polymerisationsfähige Milchsäure darstellt.

3. Verfahren nach Anspruch 1 oder 2, wobei das erste Reinigen mittels Ionenaustausch mit einer Nanofiltration kombiniert wird.

4. Verfahren nach einem der vorhergehenden Ansprüche 1 bis 3, wobei das Abtrennen der Biomasse und eventuell vorhandener Feststoffe aus der Fermentationsbrühe in der ersten Stufe durch eine Precoat- und/oder eine Mikrofiltration und/oder eine Zentrifugation erfolgt.

5. Verfahren nach einem der vorhergehenden Ansprüche 1 bis 4, wobei das Abtrennen der Biomasse aus der Fermentationsbrühe ohne thermische Inaktivierung erfolgt.

6. Verfahren nach einem der vorhergehenden Ansprüche 1 bis 5, wobei die Zeit zwischen Beendigung der Fermentation und der Abtrennung der Biomasse kleiner 2 h, vorzugsweise kleiner 1 h beträgt.

7. Verfahren nach einem der vorhergehenden Ansprüche 1 bis 6, wobei das Abtrennen der Biomasse und eventuell vorhandener Feststoffe aus der Fermentationsbrühe in der zweiten Stufe durch eine ein- oder zweistufige Ultrafiltration erfolgt und Membranen mit einer Trenngrenze von $\leq$ 10 kDa eingesetzt werden.

8. Verfahren nach Anspruch 7, wobei ein bei der Ultrafiltration entstehendes Retentat zur ersten Stufe zurückgeführt wird und das entstehende Permeat den nachfolgenden Verfahrensschritten zugeführt wird.

9. Verfahren nach einem der vorhergehenden Ansprüche 1 bis 8, wobei eine Ansäuerung in zwei Schritten erfolgt, in einem 1. Schritt die Fermentationsbrühe aus der ersten Stufe des ersten Verfahrensschrittes mit konzentrierter Schwefelsäure auf einen pH-Wert von 4,4 bis 4,6 angesäuert wird und in einem 2. Schritt das Permeat der Ultrafiltration aus der zweiten Stufe des ersten Verfahrensschrittes mit konzentrierter Schwefelsäure auf einen pH-Wert von 2,0 bis 2,4 angesäuert wird, so dass das in der gereinigten Fermentationslösung enthaltene Salz der Milchsäure in Milchsäure überführt wird und im stöchiometrischen Verhältnis Salz entsteht, und wobei die Temperatur des angesäuerten Permeates der Ultrafiltration in einem Bereich zwischen 30°C bis 60°C, und vorzugsweise in einem Bereich zwischen 30 bis 40°C gehalten wird.

10. Verfahren nach einem der vorhergehenden Ansprüche 1 bis 9, wobei das Permeat der Ultrafiltration mittels der SMB in einen die Hauptmenge der Milchsäure enthaltenden Extrakt und einen die Hauptmenge Ammoniumsulfat sowie geringe Mengen an Begleitsalzen wie Phosphate, Nitrate und Chloride enthaltendes Raffinat getrennt wird, wobei

- die Zugabe des Permeates der Ultrafiltration und eines Eluents kontinuierlich in einem Verhältnis Permeat: Eluent von 1 : 1,5 bis 1 : 2,5 erfolgt,
- der die Milchsäure enthaltende Extrakt, aufweisend einen Gehalt an Milchsäure $\geq$1 g/l, und das Raffinat getrennt voneinander gesammelt werden,

- der Wirkungsgrad der Gewinnung von Milchsäure aus dem Permeat der Ultrafiltration ≥ 95% beträgt.

11. Verfahren nach einem der vorhergehenden Ansprüche 1 bis 10, wobei beim Reinigen mittels lonenaustausch eine Reinigung von anionischen und kationischen Unreinheiten durch Kationenaustauscher und/oder Anionenaustauscher durchgeführt wird.

12. Verfahren nach einem der Ansprüche 1 bis 11, wobei bei der ersten Aufkonzentrierung eine Aufkonzentrierung der Milchsäure auf 45 bis 55 Ma-% erfolgt.

13. Verfahren nach einem der Ansprüche 1 bis 12, wobei bei der zweiten Aufkonzentrierung eine Aufkonzentrierung der Milchsäure auf ≥ 80 Ma-% erfolgt.

14. Verwendung der gemäß einem der Ansprüche 1 bis 13 hergestellten polymerisationsfähigen Milchsäure zur Herstellung von Polylactiden, wobei die Milchsäure eine Dilactidausbeute von >90 % und eine Racemisierung von <5 % aufweist.

15. Verwendung nach Anspruch 14, wobei die Herstellung von Polylactiden die nachfolgenden Verfahrensschritte umfasst:

  - Aufkonzentrieren der polymerisationsfähigen Milchsäure auf ≥ 98 Ma-%,
  - Präkondensation der aufkonzentrierten Milchsäure auf eine mittlere Molmasse von 500 bis 2000 g/mol,
  - cyclisierende Depolymeristion der präkondensierten Milchsäure, wobei das Lactid generiert wird,
  - Reinigen des Lactids,
  - Ringöffnungspolymerisation des Lactids, wobei Polylactid generiert wird,
  - Entmonomerisierung des Polylactids, und
  - Granulierung des Polylactids zu Polylactid Granulat und
  - Kristallisation des Polylactid Granulats.

16. Verfahren zur Herstellung von Polylactiden aus polymerisationsfähiger Milchsäure umfassend die nachfolgenden Verfahrensschritte:

  - Herstellen einer polymerisationsfähigen Milchsäure nach dem in einem der Ansprüche 1 bis 13 definierten Verfahren,wobei die Milchsäure eine Dilactidausbeute von >90 % und eine Racemisierung von <5 % aufweist,
  - Aufkonzentrieren der polymerisationsfähigen Milchsäure auf ≥ 98 Ma-%,
  - Präkondensation der aufkonzentrierten polymerisationsfähigen Milchsäure auf eine mittlere Molmasse von 500 bis 2000 g/mol,
  - cyclisierende Depolymeristion der präkondensierten Milchsäure, wobei das Lactid generiert wird,
  - Reinigen des Lactids,
  - Ringöffnungspolymerisation des Lactids, wobei Polylactid generiert wird,
  - Entmonomerisierung des Polylactids,
  - Granulierung des Polylactids zu Polylactid Granulat und Kristallisation des Polylactid Granulats.

**Claims**

1. Method for producing polymerizable lactic acid from fermentation broths, comprising the method steps of:

  - removing the biomass and possible solids present from the fermentation broth in at least two successive stages,
  - lowering the pH to a value of 2.2-2.4 by adding and mixing concentrated sulphuric acid into the lactic acid solution from the biomass-free fermentation broth,
  - separating the lactic acid solution from the biomass-free fermentation broth by simulated moving bed chromatography (SMB),
  - purifying the lactic acid solution separated by means of a first ion exchange step,
  - concentrating the lactic acid solution purified in the first ion exchange step by means of a first one-stage or multi-stage evaporation stage,
  - further purifying the pre-concentrated and pre-purified lactic acid solution by means of a second ion exchange step,
  - concentrating the lactic acid solution purified in the second ion exchange step by means of a second one-stage or multi-stage evaporation stage, such that

the lactic acid comprises a dilactide yield of > 90% and racemization of < 5%;
wherein the polymerizable lactic acid is produced without distillation.

2. Method according to Claim 1, wherein the lactic acid is subjected to a test method comprising the method steps of:

- polycondensing the lactic acid to give a prepolymer,
- depolymerizing the prepolymer to give the dilactide, and
- determining the dilactide yield and racemization, wherein lactic acid which comprises a dilactide yield of > 90%, preferably > 93% and racemization of < 5%, preferably < 3%,

is a polymerizable lactic acid.

3. Method according to Claim 1 or 2, wherein the first purification by means of ion exchange is combined with nanofiltration.

4. Method according to any of preceding Claims 1 to 3, wherein the biomass and possible solids present are removed from the fermentation broth in the first stage by a precoat filtration and/or a microfiltration and/or a centrifugation.

5. Method according to any of preceding Claims 1 to 4, wherein the biomass is removed from the fermentation broth without thermal inactivation.

6. Method according to any of preceding Claims 1 to 5, wherein the time between completion of the fermentation and the removal of the biomass is less than 2 h, preferably less than 1 h.

7. Method according to any of preceding Claims 1 to 6, wherein the biomass and possible solids present are removed from the fermentation broth in the second stage by a single-stage or two-stage ultrafiltration and membranes having a cut-off of $\leq$ 10 kDa are used.

8. Method according to Claim 7, wherein a retentate resulting from the ultrafiltration is recycled to the first stage and the resulting permeate is fed to the subsequent method steps.

9. Method according to any of preceding Claims 1 to 8, wherein acidification is carried out in two steps, in a $1^{st}$ step the fermentation broth from the first stage of the first method step is acidified with concentrated sulphuric acid to a pH of 4.4 to 4.6 and in a $2^{nd}$ step the permeate of the ultrafiltration from the second stage of the first method step is acidified with concentrated sulphuric acid to a pH of 2.0 to 2.4, such that the salt of the lactic acid present in the purified fermentation solution is converted into lactic acid and salt is formed in the stoichiometric ratio, and wherein the temperature of the acidified permeate of the ultrafiltration is maintained in a range between 30°C and 60°C, and preferably in a range between 30 and 40°C.

10. Method according to any of preceding Claims 1 to 9, wherein the permeate of the ultrafiltration is separated by means of SMB into an extract comprising the major amount of the lactic acid, and a raffinate comprising the major amount of ammonium sulphate and also minor amounts of accompanying salts, such as phosphates, nitrates and chlorides, wherein

- the permeate of the ultrafiltration and an eluent are added continuously in a ratio of permeate:eluent of from 1:1.5 to 1:2.5,
- the extract comprising the lactic acid, comprising a lactic acid content of $\geq$ 1 g/l, and the raffinate are collected separately from each other,
- the efficiency of the recovery of lactic acid from the permeate of the ultrafiltration is $\geq$ 95%.

11. Method according to any of preceding Claims 1 to 10, wherein on purifying by means of ion exchange, purification of anionic and cationic impurities is carried out by cation exchangers and/or anion exchangers.

12. Method according to any of Claims 1 to 11, wherein in the first concentration, the lactic acid is concentrated to 45 to 55 mass%.

**13.** Method according to any of Claims 1 to 12, wherein in the second concentration, the lactic acid is concentrated to ≥ 80 mass%.

**14.** Use of the polymerizable lactic acid produced according to any of Claims 1 to 13 for producing polylactides, wherein the lactic acid comprises a dilactide yield of > 90% and racemization of < 5%.

**15.** Use according to Claim 14, wherein the production of polylactides comprises the following method steps:

- concentrating the polymerizable lactic acid to ≥ 98 mass%,
- pre-condensing the concentrated lactic acid to an average molar mass of 500 to 2000 g/mol,
- performing cyclizing depolymerization of the pre-condensed lactic acid, wherein the lactide is generated,
- purifying the lactide,
- performing ring-opening polymerization of the lactide, wherein polylactide is generated,
- demonomerizing the polylactide, and
- granulating the polylactide to give polylactide granules and
- crystallizing the polylactide granules.

**16.** Method for producing polylactides from polymerizable lactic acid comprising the following method steps:

- producing a polymerizable lactic acid according to the method defined in any of Claims 1 to 13, wherein the lactic acid comprises a dilactide yield of > 90% and racemization of < 5%,
- concentrating the polymerizable lactic acid to ≥ 98 mass%,
- pre-condensing the concentrated polymerizable lactic acid to an average molar mass of 500 to 2000 g/mol,
- performing cyclizing depolymerization of the pre-condensed lactic acid, wherein the lactide is generated,
- purifying the lactide,
- performing ring-opening polymerization of the lactide, wherein polylactide is generated,
- demonomerizing the polylactide,
- granulating the polylactide to give polylactide granules and

crystallizing the polylactide granules.

**Revendications**

**1.** Procédé pour la préparation d'acide lactique apte à la polymérisation à partir de bouillons de fermentation, comprenant les étapes de procédé :

- séparer la biomasse et les solides éventuellement présents du bouillon de fermentation en au moins deux étages consécutifs,
- diminuer la valeur du pH à une valeur de 2,2-2,4 par addition et mélange d'acide sulfurique concentré dans la solution d'acide lactique provenant du bouillon de fermentation exempt de biomasse,
- séparer la solution d'acide lactique du bouillon de fermentation exempt de biomasse par chromatographie sur lit mobile simulé (Simulated Moving Bed Chromatography - SMB),
- purifier la solution d'acide lactique séparée au moyen d'une première étape d'échange ionique,
- concentrer la solution d'acide lactique purifiée dans la première étape d'échange ionique au moyen d'un premier étage d'évaporation à un ou plusieurs étages,
- purifier davantage la solution d'acide lactique préconcentrée et prépurifiée au moyen d'une deuxième étape d'échange ionique,
- concentrer la solution d'acide lactique purifiée dans la deuxième étape d'échange ionique au moyen d'un deuxième étage d'évaporation à un ou plusieurs étages, de manière telle que l'acide lactique présente un rendement en dilactide > 90% et une racémisation < 5% ;

la préparation de l'acide lactique apte à la polymérisation ayant lieu sans distillation.

**2.** Procédé selon la revendication 1, l'acide lactique étant soumis à un procédé test, comprenant les étapes de procédé :

- polycondenser l'acide lactique en un prépolymère,
- dépolymériser le prépolymère en dilactide et

- déterminer le rendement en dilactide et la racémisation,

l'acide lactique qui présente un rendement en dilactide > 90%, de préférence > 93% et une racémisation < 5%, de préférence < 3% représentant un acide lactique apte à la polymérisation.

3. Procédé selon la revendication 1 ou 2, la première purification au moyen d'un échange ionique étant combinée à une nanofiltration.

4. Procédé selon l'une quelconque des revendications précédentes 1 à 3, la séparation de la biomasse et des solides éventuellement présents du bouillon de fermentation dans le premier étage ayant lieu par une filtration par précouches et/ou une microfiltration et/ou une centrifugation.

5. Procédé selon l'une quelconque des revendications précédentes 1 à 4, la séparation de la biomasse du bouillon de fermentation ayant lieu sans inactivation thermique.

6. Procédé selon l'une quelconque des revendications précédentes 1 à 5, le temps entre la fin de la fermentation et la séparation de la biomasse étant inférieur à 2 h, de préférence inférieur à 1 h.

7. Procédé selon l'une quelconque des revendications précédentes 1 à 6, la séparation de la biomasse et des solides éventuellement présents du bouillon de fermentation dans le deuxième étage ayant lieu par une ultrafiltration à un ou deux étages et des membranes présentant une limite de séparation $\leq$ 10 kDa étant utilisées.

8. Procédé selon la revendication 7, un retentat formé lors de l'ultrafiltration étant recyclé vers le premier étage et le perméat formé étant introduit dans les étapes de procédé suivantes.

9. Procédé selon l'une quelconque des revendications précédentes 1 à 8, une acidification en deux étapes ayant lieu, dans une première étape, le bouillon de fermentation provenant du premier étage de la première étape de procédé est acidifié avec de l'acide sulfurique concentré à une valeur de pH de 4,4 à 4,6 et, dans une deuxième étape, le perméat de l'ultrafiltration du deuxième étage de la première étape de procédé est acidifié avec de l'acide sulfurique concentré à une valeur de pH de 2,0 à 2,4, de manière telle que le sel de l'acide lactique contenu dans la solution de fermentation purifiée est transformé en acide lactique et qu'un sel se forme dans un rapport stoechiométrique, et la température du perméat acidifié de l'ultrafiltration étant maintenue dans une plage entre 30°C à 60°C et de préférence dans une plage entre 30 à 40°C.

10. Procédé selon l'une quelconque des revendications précédentes 1 à 9, le perméat de l'ultrafiltration étant séparé au moyen de la SMB en un extrait contenant la quantité principale de l'acide lactique et en un raffinat contenant la quantité principale de sulfate d'ammonium ainsi que de petites quantités de sels secondaires, tels que des phosphates, des nitrates et des chlorures,

- l'addition du perméat de l'ultrafiltration et d'un éluant ayant lieu en continu dans un rapport perméat:éluant de 1:1,5 à 1:2,5,
- l'extrait contenant l'acide lactique, présentant une teneur en acide lactique $\geq$ 1 g/l, et le raffinat étant récupérés séparément l'un de l'autre,
- le degré d'efficacité de la production d'acide lactique à partir du perméat de l'ultrafiltration étant $\geq$ 95%.

11. Procédé selon l'une quelconque des revendications précédentes 1 à 10, une purification des impuretés anioniques et cationiques étant réalisée par des échangeurs cationiques et/ou des échangeurs anioniques lors de la purification au moyen d'un échange ionique.

12. Procédé selon l'une quelconque des revendications 1 à 11, une concentration de l'acide lactique à 45 jusqu'à 55% en masse ayant lieu lors de la première concentration.

13. Procédé selon l'une quelconque des revendications 1 à 12, une concentration de l'acide lactique à $\geq$ 80% en masse ayant lieu lors de la deuxième concentration.

14. Utilisation de l'acide lactique apte à la polymérisation préparé selon l'une quelconque des revendications 1 à 13 pour la préparation de polylactides, l'acide lactique présentant un rendement en dilactide > 90% et une racémisation < 5%.

**15.** Utilisation selon la revendication 14, la préparation de polylactides comprenant les étapes de procédé suivantes :

- concentrer l'acide lactique apte à la polymérisation à ≥ 98% en masse,
- précondenser l'acide lactique concentré à une masse molaire moyenne de 500 à 2000 g/mole,
- dépolymériser de manière cyclisante l'acide lactique précondensé, le lactide étant généré,
- purifier le lactide,
- polymériser le lactide avec ouverture du cycle, le polylactide étant généré,
- démonomériser le polylactide et
- granuler le polylactide en granulat de polylactide et
- cristalliser le granulat de polylactide.

**16.** Procédé pour la préparation de polylactides à partir d'acide lactique apte à la polymérisation comprenant les étapes de procédé suivantes :

- préparer un acide lactique apte à la polymérisation selon le procédé défini dans l'une quelconque des revendications 1 à 13, l'acide lactique présentant un rendement en dilactide > 90% et une racémisation < 5%,
- concentrer l'acide lactique apte à la polymérisation à ≥ 98% en masse,
- précondenser l'acide lactique apte à la polymérisation, concentré, à une masse molaire moyenne de 500 à 2000 g/mole,
- dépolymériser de manière cyclisante l'acide lactique précondensé, le lactide étant généré,
- purifier le lactide,
- polymériser le lactide avec ouverture du cycle, le polylactide étant généré,
- démonomériser le polylactide,
- granuler le polylactide en granulat de polylactide et
- cristalliser le granulat de polylactide.

Figur. 1

Figur 2:

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0986532 B2
- DE 102007045701 B3
- DE 69815369 T2
- DE 102009019248 A1
- WO 2006124633 A1
- EP 2374895 A1
- WO 2009030397 A1

**In der Beschreibung aufgeführte Nicht-Patentliteratur**

- **J. DAHLMANN et al.** *British Polymer Journal,* 1990, vol. 23, 235.240